(19) Europäisches Patentamt
European Patent Office
Office européen des brevets

(11) EP 3 711 749 A1

(12) EUROPEAN PATENT APPLICATION

(43) Date of publication:
23.09.2020 Bulletin 2020/39

(51) Int Cl.:
A61K 9/127 (2006.01)    A61K 38/00 (2006.01)
A61K 9/51 (2006.01)

(21) Application number: 19163780.0

(22) Date of filing: 19.03.2019

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: Polymun Scientific Immunbiologische
Forschung GmbH
3400 Klostemeuburg (AT)

(72) Inventors:
• Wagner, Andreas
  1130 Wien (AT)
• Aichinger, Frances-Dee
  1110 Wien (AT)
• Loidl, Harald
  1020 Wien (AT)

(74) Representative: Bogensberger, Burkhard
Bogensberger Patent- und Markenbüro
Fallsgasse 7
9492 Eschen (LI)

(54) METHOD OF MAKING LIPID NANOPARTICLES

(57)    What is described is a method of producing lipid nanoparticles having an average diameter of less than 100 nm, comprising pumping an aqueous buffer solution through a 1st tube by a 1st HPLC pump, pumping an organic lipid solution through a 2nd tube by a 2nd HPLC pump, wherein the 2nd tube intersects the 1st tube perpendicularly within a mixing module, and wherein the organic lipid solution is mixed with the aqueous solution in a turbulent flow within the mixing module.

Fig. 2

## Description

### FIELD OF THE INVENTION

The present invention is in the field of liposome technology and relates to an improved method of making lipid nanoparticles, typically for use as drug carrier systems.

### STATE OF THE ART

Lipids are used as materials for drug delivery owing to their ability to form lipid nanoparticles that encapsulate the desired drug for delivery to target cells upon parenteral administration.

Different methods of producing lipid-encapsulated drug nanoparticles are known.

WO 01/05373 discloses techniques for preparing lipid-encapsulated RNA nanoparticles using an ethanol injection-type process with a static mixer that provides a turbulent environment, which after vesicle formation are combined with a therapeutic molecule. US 2004/0142025 discloses techniques for forming a lipid-encapsulated RNA nanoparticles using non-turbulent mixing and a series of sequential stepwise dilutions. US 6,843,942 discloses a method of forming by non-turbulent mixing, by spraying lipids in an organic solution pipe through an orifice into nucleic acids in an aqueous solution flowing past the orifice. US 9005654 discloses encapsulating siRNA in a lipid nanoparticle (LNP) using turbulent mixing, whereby lipids and RNA as opposing flows enter a T-shaped mixing chamber from opposite arms at about equal rates to produce a 45-60% ethanol solution comprising vesicles, which are collected and then further diluted (direct dilution method). US 9404127 discloses that a majority of the LNP produced by the direct dilution method have non-lamellar morphology, i.e., a non-bilayer structure.

A need exists to improve the process and apparatus for formulating lipid nanoparticles for drug delivery, including the ability to reliably scale up to large amounts of production while optimizing for particle size and homogeneity.

### BRIEF DESCRIPTION OF THE INVENTION

Disclosed herein is a method of producing lipid nanoparticles comprising the steps pumping an aqueous solution optionally comprising a desired water-soluble drug through a 1st tube having an inner diameter (ID) of at least 0.1 ";

pumping an ethanol solution comprising lipids and optionally a hydrophobic drug through a 2nd tube having an ID of at least 0.005" at one third the flow rate of the 1st solution through the 1st tube, wherein the lipids comprise lipids selected from the group consisting of cationic lipids, ionizable cationic lipids, anionic lipids, ionizable anionic lipds, and neutral lipids; and

mixing the ethanol solution with the aqueous solution by introducing the ethanol solution into the aqueous solution flowing past the 1st tube within a mixing module consisting of the 2nd tube perpendicularly joined to the 1st tube to produce an output solution;

wherein the mixing produces an output comprising a turbulent flow of the mixed solution comprising spontaneously formed liposomal nanoparticles in 25% ethanol, and wherein the lipid nanoparticle has a bilayer structure.

In one embodiment the combined flow rate is at least 200 ml/min. In another embodiment, the combined flow has a Reynolds number of at least 2000.

In one embodiment, the aqueous solution is pumped through the 1st tube by a 1st HPLC pump, preferably with a back pressure of at least 10 psi, 25 psi, 50 psi, 75 psi, or 100 psi. Preferably, the 1st tube has an ID of 0.132", and the aqueous solution is pumped at a flow rate of at least 30 ml/min, 45 ml/min, 60 ml/min, 75 ml/min, 90 ml/min, 105 ml/min, 120 ml/min, 150 ml/min, 225 ml/min, 262.5 ml/min, 300 ml/min, or 450 ml/min.

In another embodiment, the ethanol solution is pumped through the 2nd tube by a 2nd HPLC pump, preferably with a back pressure of at least 40 psi, 80 psi, 150 psi, 300 psi, or 400 psi. Preferably, the 2nd tube has an ID of 0.007", 0.01", or 0.02"; and the ethanol solution is pumped at a flow rate of at least 10 ml/min, 15 ml/min, 20 ml/min, 25 ml/min, 30 ml/min, 35 ml/min, 40 ml/min, 50 ml/min, 60 ml/min, or 75 ml/min, 87.5 ml/min, 100 ml/min, or 150 ml/min.

In one embodiment, the mixing module consists of the 2nd tube mounted perpendicularly on the 1st tube, wherein the 1st tube has an opening through a wall, wherein the opening is the size of the outside diameter of the 2nd tube, and wherein the 2nd tube is fitted over the opening to permit continuous movement of the 2nd solution in the 2nd tube into the 1st solution in the 1st tube. The mixing module preferably consists of stainless steel tubing.

In one embodiment, the method disclosed herein further comprises pumping a dilution buffer through a 3rd tube, and mixing the dilution buffer with the output solution by introducing the dilution buffer to the output solution in the region of a Y connector to produce a diluted output solution. Preferably, the dilution buffer comprises 15 mM Tris, 50 mM NaCl, 9% sucrose, pH 7.5; 10mM Tris, 50mM NaCl, 9% sucrose, pH 7.5; 50mM phosphate, pH 6.0; 20mM HEPES, 50mM NaCl, 9% sucrose, pH 7.4; or 50mM HEPES, 50mM NaCl, 9% sucrose, pH 7.4. Preferably, the diluted output solution comprises 6.25% ethanol; 8.25% ethanol; or 12.5% ethanol.

In one embodiment, the Y-tube is at an angle of about 45°. Preferably, the Y connector consists of polyether ether ketone. In one embodiment, the dilution buffer is pumped through the 3rd tube at a flow rate of 400-900 mL/min, and the 3rd tube has an ID of 0.25".

What is also disclosed herein are lipid nanoparticles including lipid-encapsulated drug nanoparticles produced by the method disclosed herein.

What is also disclosed is a lipid nanoparticle optionally comprising a desired drug, produced by a process comprising the steps of pumping an aqueous solution optionally comprising a desired water-soluble drug through a 1st tube having an inner diameter (ID) of at least 0.1"; pumping an ethanol solution comprising lipids and optionally a desired hydrophobic drug through a 2nd tube having an ID of at least 0.005" at one third the flow rate of the 1st solution through the 1st tube, wherein the lipids comprise a lipid selected from the group consisting of cationic lipids, ionizable cationic lipids, anionic lipids, ionizable anionic lipds, and neutral lipids; and mixing the ethanol solution with the aqueous solution by introducing the ethanol solution into the aqueous solution in the 1st tube flowing past the 2nd tube within a mixing module consisting of the 2nd tube perpendicularly joined to the 1st tube, wherein the mixing produces an output which comprises the aqueous phase and the lipids in 25% ethanol in a turbulent flow. The lipid nanoparticles produced by the process disclosed herein have a bilayer structure, i.e., a lamellar morphology. Preferably, the lipid nanoparticles have an average particle size less than 70 nm, 80 nm, 90 nm, or 100 nm; a polydispersity index (PDI) less than 0.09, 0.07, or 0.05.

What is also disclosed herein is an apparatus for producing lipid nanoparticles optionally comprising a desired drug, comprising a 1st tube having an ID of at least 0.1" connected at one end to a 1st HPLC pump and at the other end to a mixing module, wherein the 1st HPLC pump is configured to pump an aqueous solution optionally comprising a desired water-soluble drug through the 1st tube at a flow rate of at least 150 ml/min; a 1st reservoir connected to the 1st HPLC pump, wherein the 1st reservoir contains the aqueous solution; a 2nd tube having an ID of at least 0.005" connected at one end to a 2nd HPLC pump and at the other end to the mixing module, wherein the 2nd HPLC pump is configured to pump an ethanol solution comprising lipid and optionally a desired hydrophobic drug through the 2nd tube at a flow rate greater than 50 ml/min, and wherein the 2nd tube is perpendicularly joined to the 1st tube at the mixing module; a 2nd reservoir connected to the 2nd HPLC pump, wherein the 2nd reservoir contains the ethanol solution; wherein the apparatus is configured to mix the ethanol solution with the aqueous solution by introducing the ethanol solution into the aqueous solution in a region within the mixing module to produce an output solution; and wherein the flow of the output solution produces turbulence.

The apparatus disclosed herein preferably has the mixing module where the 2nd tube extends through a wall of the 1st tube and partly into the interior of the 1st tube; or the 2nd tube extends up to a wall of the 1st tube and joins the 1st tube.

## DESCRIPTION OF THE DRAWINGS

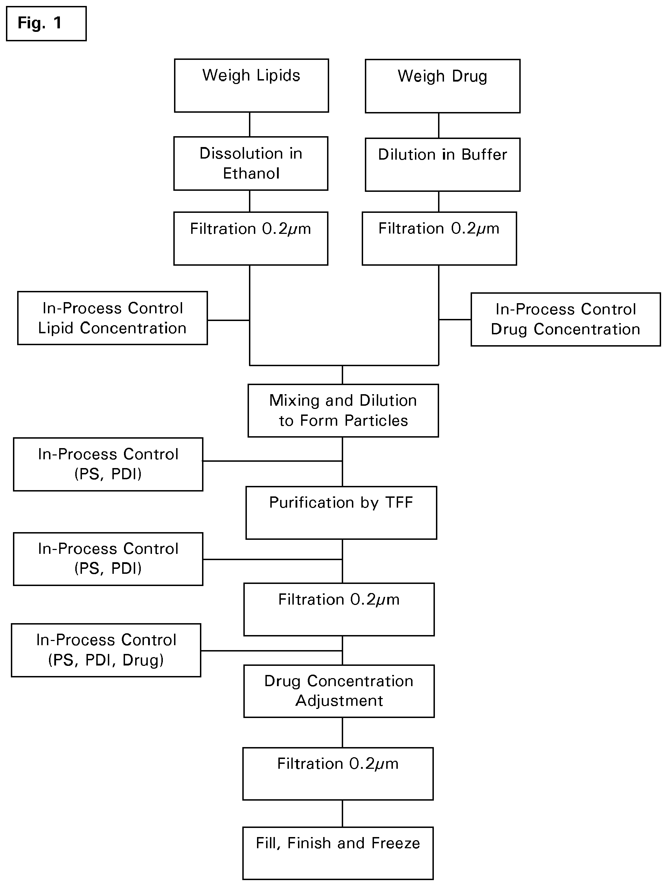

**FIG. 1** shows a flow chart diagram for one embodiment of a process of producing lipid nanoparticles. Lipids are dissolved in ethanol optionally together with a hydrophobic drug, a desired water soluble drug - as the case may be - is dissolved in an aqueous acidic buffer (e.g. citrate buffer), both solutions are filter sterilized. The solutions are mixed by the process described herein to form particles, which are analyzed for PDI and particle size (PS). The particles are concentrated and purified by tangential flow filtration (TFF) to remove ethanol and unbound drug, and PDI and PS is again monitored. The concentration of the particles is then adjusted to a desired concentration. The particles are filter sterilized, filled, finished, and frozen.

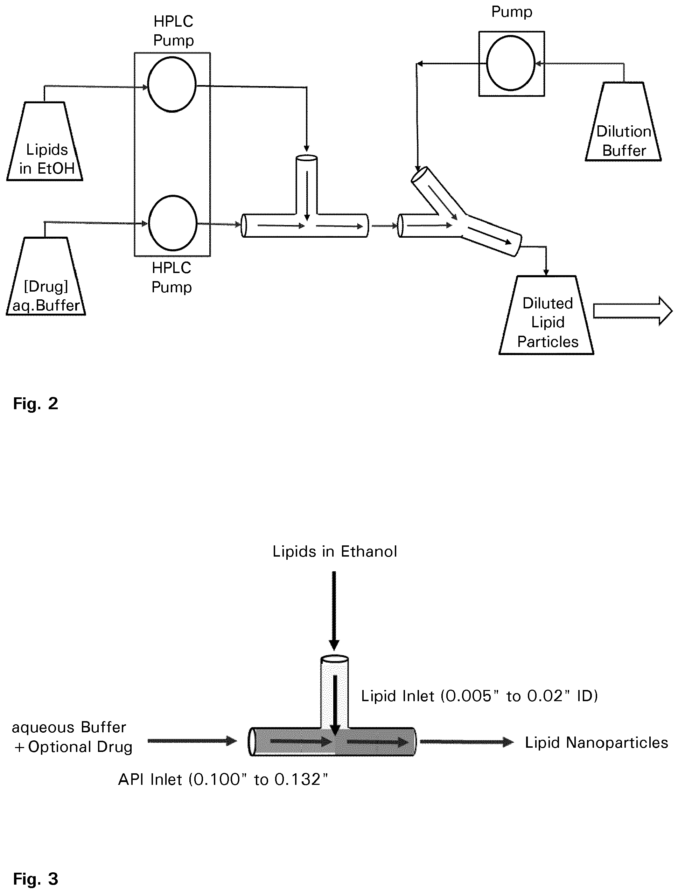

**FIG. 2** shows the apparatus for producing lipid nanoparticles. The aqueous solution is transported by an HPLC pump through tubing comprising regions of 0.03" ID PEEK tubing, 0.05" ID PEEK connector, 0.0625 " silicon tubing, and 0.122" ID silicon tubing, and 0.132" ID stainless steel. The organic solution comprising lipids is transported by an HPLC pump through tubing, e.g., comprising regions of 0.03" ID PEEK tubing, 0.02" ID PEEK connector, 0.01" ID stainless steel. The organic solution is pumped into the aqueous solution at a 90 degree angle in the mixing area. The 0.122" ID silicon tubing transports the mixed phases from the outlet to a 0.25" ID polypropylene tubing which merges at a 45 degree angle with dilution buffer in the dilution area, and the diluted particles are collected in a stainless steel-jacketed vessel maintained at 15-20 degrees C. The particles are further processed by tangential flow filtration using a peristaltic, diaphragm or centrifugal pump.

**FIG. 3** shows the mixing module in more detail. The aqeous buffer is transported through an input arm of a 1st (e.g., 0.100-0.132" ID) stainless steel tube. The lipids in ethanol are transported through a 2nd (e.g., 0.005-0.010" ID) stainless steel tube that is perpendicularly attached to the 1st tube. A hole in the wall of the first tube allows transport of the lipid phase from the 2nd tube to the interior of the 1st tube, lipid nanoparticles or lipid-encapsulated drug nanoparticles resulting from a mixing exit through an output arm of the 1st tube.

## DETAILED DESCRIPTION

Applicants have discovered a process of mixing lipid and aqueous buffer using turbulent flow to produce lipid nanoparticles, or more precisely, nearly monodisperse particles of less than 100 nm having a lamellar morphology, i.e., including a bilayer structure. The process is scalable.

### Definitions

"Turbulence" is defined herein in terms of the geometry of the mixing area, and the flow rates of the aqueous solution optionally comprising a water-soluble drug and the ethanol solution comprising a lipid mixture and optionally a hydrophobic drug. During mixing the Reynolds number is at least 2000, in which the Reynolds number Re is defined as:

$$Re = \frac{DV\rho}{\mu}$$

wherein: $\rho$ is the density of a solution comprising 0 to 25% ethanol in water (g/cm$^3$), V is the velocity of the solution with respect to the tubing (cm/s), D is the ID of the tubing (cm), $\mu$ is the dynamic viscosity of the solution (Pa·s). Tilton, Fluid and Particle Dynamics, PERRY'S CHEMICAL ENGINEERS' HANDBOOK (Green ed., 8th ed. 2008), at page 6-51. Transitions to turbulent flow occur with Reynolds number Re in the range of 2000 to 2500 (Tilton, at p6-14). For example, at 300 ml/min flow rate in tubing with ID = 0.132", Re is 2,100 (D = 0.30 cm, V=70 cm/sec, $\rho$=96 g/cm$^3$, and $\mu$=0.01 gm/cm$^3$ sec (Poling, Physical and Chemical Data, in PERRY'S, at p2-117 and p2-448, respectively),. Taking the velocity profile of the tube into account, centerline velocity, $v$ as a function of radial position r in a circular pipe of radius R (D/2) is defined as

$$v = 2V(1\text{-}r^2/R^2)$$

where the maximum velocity is twice the effective velocity for a parabolic profile (Tilton, at p6-11). For example at a flow rate of 300 ml/min, the Reynolds number in the center of the tube is 4200. For this reason, it is possible to determine that a flow rate in which turbulence is produced at least in the center of the tubing, and to a considerable extent toward the walls.

"Aqueous solution" refers to a composition comprising in whole, or in part, water. "Organic lipid solution" refers to a composition comprising in whole, or in part, an organic solvent having a lipid. The organic lipid solution preferably comprises an alkanol, most preferably ethanol.

"Lipid" refers to a group of organic compounds that are esters of fatty acids and are characterized by being insoluble in water but soluble in many organic solvents, e.g., fats, oils, waxes, phospholipids, glycolipids, and steroids.

"Amphipathic lipid" comprises a lipid in which hydrophilic characteristics derive from the presence of polar or charged groups such as carbohydrates, phosphato, carboxylic, sulfato, amino, sulfhydryl, nitro, hydroxy and other like groups, and hydrophobic characteristics can be conferred by the inclusion of polar groups that include, but are not limited to, long chain saturated and unsaturated aliphatic hydrocarbon groups and such groups substituted by one or more aromatic, cycloaliphatic or heterocyclic group(s). Examples include phospholipids, aminolipids and sphingolipids. Phospholipids include phosphatidylcholine, phosphatidylethanolamine, phosphatidylserine, phosphatidylinositol, phosphatidic acid, palmitoyloleoyl phosphatidylcholine, lysophosphatidylcholine, lysophosphatidylethanolamine, dipalmitoylphosphatidylcholine, dioleoylphosphatidylcholine, distearoylphosphatidylcholine or dilinoleoylphosphatidylcholine. Amphipathic lipids also can lack phosphorus, such as sphingolipids, glycosphingolipid families, diacylglycerols and $\beta$-acyloxyacids.

"Anionic lipid" is any lipid that is negatively charged at physiological pH, including phosphatidylglycerol, cardiolipin, diacylphosphatidylserine, diacylphosphatidic acid, N-dodecanoyl phosphatidylethanolamines, N-succinyl phosphatidylethanolamines, N-glutarylphosphatidylethanolamines, lysylphosphatidylglycerols, and other anionic modifying groups joined to neutral lipids.

"Cationic lipids" carry a net positive charge at a selective pH, such as physiological pH, including N,N-dioleyl-N,N-dimethylammonium chloride ("DODAC"); N-(2,3-dioleyloxypropyl)-N,N,N-trimethylammonium chloride ("DOTMA"); N,N-distearyl-N,N-dimethylammonium bromide ("DDAB"); N-(2,3-dioleoyloxy)propyl)-N,N,N-trimethylammonium chloride ("DOTAP"); 3-(N-(N',N'-dimethylaminoethane)-carbamoyl)cholesterol ("DC-Chol") and N-(1,2-dimyristyloxyprop-3-yl)-N,N-dimethyl-N-hydroxyethyl ammonium bromide ("DMRIE"). Additionally, a number of commercial preparations of cationic lipids are available which can be used in the present invention. These include, for example, LIPOFECTIN®, LIPOFECTAMINE®, and TRANSFECTAM®. An "ionizable cationic lipid" carries a charge that is pH-dependent. Ionizable cationic lipids include those disclosed in U.S. Patent Nos. 9,593,077, 9,365,610, 9,567,296, 9,580,711, and 9,670,152, and U.S. Patent Application Publication Nos. 2017/0190661 and 2017/0114010, incorporated herein by reference in their entirety.

"Lipid nanoparticle" is any lipid composition in the form of a liposome that can be used to deliver a compound and which comprises a lipid bilayer, either as unilamellar or multilamellar structure, in which typically a desired drug is encapsulated, optionally at least in part by ionic pairing with anionic or cationic lipids.

"Lamellar morphology" refers to a bilayer structure. The lamellar morphology, bilayer structure of the lipid particles disclosed herein can be determined using analytical techniques, e.g., by cryo-TEM images.

"Lipid-encapsulated" can refer to a lipid formulation which provides a compound with full encapsulation, partial encapsulation, or both.

**Lipid nanoparticle formation**

**FIG.** 1 is an example of a representative flow chart of the method described herein of producing lipid nanoparticles.

The geometry of the mixing layer consists of a first tube for transporting the aqueous solution having an inner diameter (ID) of greater than 0.1", preferably an ID greater than 0.132"; and a second tube for transporting the ethanol solution consisting of a ID greater than 0.005", preferably greater than 0.01"; in which the second (organic) tube intersects the first (aqueous) tube at or near a perpendicular angle. The combined flow rate during mixing is at least 200 ml/min, preferably at least 300 min.

The method described herein provides an aqueous solution optionally comprising a therapeutic water-soluble drug, e.g., prepared under Good Manufacturing Practice (GMP), solubilized in an aqueous solution comprising a buffer, e.g., citrate. The present method also provides an organic solution comprising one or more lipids, e.g., clinical grade lipids synthesized under GMP, produced by solubilizing lipid in a water-miscible organic solvent, and optionally a hydrophobic therapeutic drug. In the method described herein, the water-miscible organic solvent preferably is a lower alkanol, e.g., ethanol. Preferably, both solutions are filter sterilized and their concentrations are adjusted.

The organic lipid solution is mixed with the aqueous solution to form a lipid nanoparticle having a lamellar morphology, i.e., including a lipid bilayer. In one aspect, a water-soluble drug is encapsulated in the lipid nanoparticles along with the formation of the lamellar structure.

The method described herein is directed to continuously introducing a lipid solution into the aqueous solution in a mixing environment, preferably perpendicularly in a mixing module. The mixing dilutes the lipid solution with the aqueous solution to 20%, 22.5%, 25%, 27.5%, or 30% ethanol, preferably 25% ethanol, and causes formation of lipid nanoparticles in a turbulent flow.

After formation of the lipid nanoparticles, the mixture is continuously diluted by a buffer to 7.5%, 10%, 12.5%, or 15%, preferably to less than 12.5% ethanol, which further stabilizes the lipid nanoparticles and possibly increases encapsulation of desired drug.

The lipid nanoparticles are then concentrated typically by tangential flow filtration, preferably by hollow fiber filters. The concentrated lipid nanoparticles are subjected to an ultrafiltration step to remove the alkanol and substitute the buffer. The desired drug concentration is adjusted by dilution. The resulting formulation is filter sterilized, and filled in vials. The process will now be discussed in more detail herein below using the steps as set forth in FIG. 1.

**Lipid Solubilization**

The preferred size for lipid nanoparticles comprising a desired drug for a specific therapeutic application is about 50-200 nm in diameter, preferably, with a size distribution in which the mean size (e.g., diameter) is about 70 nm to about 150 nm, and more preferably the mean size is less than about 100 nm.

In certain embodiments, the organic solvent concentration wherein the lipids are solubilized is about 45% v/v to about 90% v/v. In certain preferred aspects, the organic solvent is a lower alkanol. Suitable lower alkanols include, e.g., methanol, ethanol, propanol, butanol, pentanol, their isomers and combinations thereof. The solvent is preferably ethanol with a volume of about 50-90% v/v. Preferably, the lipids occupy a volume of about 1 mL/g to about 5 mL/g.

The lipids are solubilized using for example, an overhead stirrer at a suitable temperature. In one aspect, the total lipid concentration of the solution is about 49.4 mg/mL. In certain preferred aspects, a water-soluble drug is included in an aqueous solution (e.g., buffer) and is diluted to a final concentration. Preferably, the final concentration is about 0.55 mg/mL in citrate buffer, with a pH of about 3.5.

**Lipid Nanoparticle Formation**

After the organic solution and the aqueous solutions are prepared, they are mixed together using the apparatus described in detail below. Briefly, the apparatus consists of a first tube for transporting the aqueous solution and a second tube for transporting the organic lipid solution, in which the second tube intersects the first tube perpendicularly within the mixing module. The two solutions are pumped through the respective tubes by separate HPLC pumps and mix in the region of the first tube perpendicularly within the mixing module. Preferably, the aqueous solution is pumped at a rate 2.0-, 2.5-, 3.0-, 3.25-, or 3.5-fold, preferably 3.0-fold, greater than the organic lipid solution. Upon mixing the two solutions in the mixing area, lipid nanoparticles are formed.

The pump speeds and the size of the first tube in the region of the mixing module provides for a mixing process that involves turbulent flow. The processes described herein for mixing of the lipid solution and the aqueous solution also provides for encapsulation of a water-soluble drug in the lipid nanoparticles formed coincident with their formation with an encapsulation efficiency depending upon the nature of the drug and the lipid composition of the lipid nanoparticles of up to 98%.

Lipid nanoparticles are typically formed at room temperature, but may also be formed at elevated temperatures according to the present invention. There are no general requirements for buffer composition. In fact, the processes and apparatus of the present invention can formulate a lipid vesicle by mixing lipid in ethanol with an aqueous solution.

In one embodiment, lipid nanoparticles form when lipids dissolved in an organic solvent, e.g., ethanol, are diluted in a stepwise manner by mixing with a buffered aqueous solution, first by mixing the aqueous and lipid streams together in the mixing module to a final concentration of organic solvent preferably between 25-40%. The resultant lipid, solvent and solute concentrations can be kept constant throughout the vesicle formation process. Following the initial formation of lipid nanoparticles, the resulting mixture is further diluted by addition of buffer, preferably to about 6.0%, 6.25%, 7.0% 7.5%, 10%, 12.5%, or 15% organic solvent, most preferably 6.25% 6.25%, 7.0% 7.5%, 10%, or 12.5%.

The continuous process described herein is fully scalable. In one aspect, lipid nanoparticles are formed having a mean diameter of less than about 80 nm, without mechanical-energy processes such as membrane extrusion, sonication or microfluidization.

**Lipid nanoparticles**

The lipid nanoparticles disclosed herein comprise a nanoparticle or a bilayer of lipid molecules.

The lipid nanoparticles typically have a mean diameter of from 30 nm to 150 nm, from 40 nm to 150 nm, from 50 nm to 150 nm, from 60 nm to 130 nm, from 70 nm to 110 nm, or from 70 to 90 nm. The drug-loaded liposomal nanoparticles described herein also typically have a lipid:drug ratio (mass/mass ratio) of from 1:1 to 100:1, from 1:1 to 50:1, from 2:1 to 25:1, from 3:1 to 20:1, from 5:1 to 15:1, or from 5:1 to 10:1, or from 10:1 to 14:1, or from 9:1 to 20:1.

In preferred embodiments, the lipid nanoparticles comprise a cationic or anionic lipid or a salt of a cationic or of an anionic lipid, a phospholipid, and a conjugated lipid that inhibits aggregation of the lipid nanoparticles (*e.g.*, one or more PEG-lipid conjugates). The lipid nanoparticles can also include cholesterol.

**Neutral Helper Lipids**

Non-limiting examples of non-cationic lipids include phospholipids such as lecithin, phosphatidylethanolamine, lysolecithin, lysophosphatidylethanolamine, phosphatidylserine, phosphatidylinositol, sphingomyelin, egg sphingomyelin (ESM), cephalin, cardiolipin, phosphatidic acid, cerebrosides, dicetylphosphate, distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoylphosphatidylethanolamine (DOPE), palmitoyloleoyl-phosphatidylcholine (POPC), palmitoyloleoyl-phosphatidylethanolamine (POPE), palmitoyloleyol-phosphatidylglycerol (POPG), dioleoyl-phosphatidylethanolamine 4-(N-maleimidomethyl)-cyclohexane-1-carboxylate (DOPE-mal), dipalmitoyl-phosphatidylethanolamine (DPPE), dimyristoylphosphatidylethanolamine (DMPE), distearoyl-phosphatidylethanolamine (DSPE), monomethyl-phosphatidylethanolamine, dimethyl-phosphatidylethanolamine, dielaidoylphosphatidylethanolamine (DEPE), stearoyloleoyl-phosphatidylethanolamine (SOPE), lysophosphatidylcholine, dilinoleoylphosphatidylcholine, and mixtures thereof. Other diacylphosphatidylcholine and diacylphosphatidylethanolamine phospholipids can also be used. The acyl groups in these lipids are preferably acyl groups derived from fatty acids having $C_{10}$-$C_{24}$ carbon chains, *e.g.*, lauroyl, myristoyl, palmitoyl, stearoyl, or oleoyl.

Additional examples of non-cationic lipids include sterols such as cholesterol and derivatives thereof. Non-limiting examples of cholesterol derivatives include polar analogues such as 5$\alpha$-cholestanol, 5$\alpha$-coprostanol, cholesteryl-(2'-hydroxy)-ethyl ether, cholesteryl-(4'- hydroxy)-butyl ether, and 6-ketocholestanol; non-polar analogues such as 5$\alpha$-cholestane, cholestenone, 5$\alpha$-cholestanone, 5$\alpha$-cholestanone, and cholesteryl decanoate; and mixtures thereof. In preferred embodiments, the cholesterol derivative is a polar analogue such as cholesteryl-(4'-hydroxy)-butyl ether.

In some embodiments, the non-cationic lipid present in lipid particles comprises or consists of a mixture of one or more phospholipids and cholesterol or a derivative thereof. In other embodiments, the non-cationic lipid present in the lipid particles comprises or consists of one or more phospholipids, *e.g.*, a cholesterol-free lipid particle formulation.

In yet other embodiments, the non-cationic lipid present in the lipid particles comprises or consists of cholesterol or a derivative thereof, *e.g.*, a phospholipid-free lipid particle formulation.

Other examples of non-cationic lipids include nonphosphorous containing lipids such as, *e.g.*, stearylamine, dodecylamine, hexadecylamine, acetyl palmitate, glycerol ricinoleate, hexadecyl stearate, isopropyl myristate, amphoteric acrylic polymers, triethanolamine-lauryl sulfate, alkyl-aryl sulfate polyethyloxylated fatty acid amides, dioctadecyldimethyl ammonium bromide, ceramide, and sphingomyelin.

The percentage of non-cationic lipid present in the lipid particles is a target amount, and the actual amount of non-cationic lipid present in the formulation may vary, for example, by $\pm$ 5 mol %.

**Lipid Conjugates**

In addition to cationic lipids, the lipid nanoparticles described herein may further comprise a lipid conjugate. The conjugated lipid is useful in that it prevents the aggregation of particles. Suitable conjugated lipids include, but are not limited to, PEG-lipid conjugates, cationic-polymer- lipid conjugates, and mixtures thereof.

In a preferred embodiment, the lipid conjugate is a PEG-lipid. PEG is a linear, water-soluble polymer of ethylene PEG repeating units with two terminal hydroxyl groups. PEGs are classified by their molecular weights.

In certain instances, the PEG can be optionally substituted by an alkyl, alkoxy, acyl, or aryl group. The PEG can be conjugated directly to the lipid or may be linked to the lipid via a linker moiety. Any linker moiety suitable for coupling the PEG to a lipid can be used including, *e.g.*, non-ester-containing linker moieties and ester-containing linker moieties. In a preferred embodiment, the linker moiety is a non-ester-containing linker moiety.

Phosphatidylethanolamines having a variety of acyl chain groups of varying chain lengths and degrees of saturation can be conjugated to PEG to form the lipid conjugate. Such phosphatidylethanolamines are commercially available, or can be isolated or synthesized using conventional techniques known to those of skill in the art. Phosphatidylethanolamines containing saturated or unsaturated fatty acids with carbon chain lengths in the range of $C_{10}$ to $C_{20}$ are preferred. Phosphatidylethanolamines with mono- or di-unsaturated fatty acids and mixtures of saturated and unsaturated fatty acids can also be used. Suitable phosphatidylethanolamines include, but are not limited to, dimyristoyl-phosphatidylethanolamine (DMPE), dipalmitoyl-phosphatidylethanolamine (DPPE), dioleoyl-phosphatidylethanolamine (DOPE), and distearoyl-phosphatidylethanolamine (DSPE).

The term "diacylglycerol" or "DAG" includes a compound having 2 fatty acyl chains, $R^1$ and $R^2$, both of which have independently between 2 and 30 carbons bonded to the 1- and 2-position of glycerol by ester linkages. The acyl groups can be saturated or have varying degrees of unsaturation. Suitable acyl groups include, but are not limited to, lauroyl ($C_{12}$), myristoyl ($C_{14}$), palmitoyl ($C_{16}$), stearoyl ($C_{18}$), and icosoyl ($C_{20}$). In preferred embodiments, $R^1$ and $R^2$ are the same, *i.e.*, $R^1$ and $R^2$ are both myristoyl (*i.e.*, dimyristoyl), $R^1$ and $R^2$ are both stearoyl (*i.e.*, distearoyl).

The term "dialkyloxypropyl" or "DAA" includes a compound having 2 alkyl chains, $R^3$ and $R^4$, both of which have independently between 2 and 30 carbons. The alkyl groups can be saturated or have varying degrees of unsaturation.

In addition to the foregoing, other hydrophilic polymers can be used in place of PEG. Examples of suitable polymers that can be used in place of PEG include, but are not limited to, polyvinylpyrrolidone, polymethyloxazoline, polyethyloxazoline, polyhydroxypropyl methacrylamide, polymethacrylamide and polydimethylacrylamide, polylactic acid, polyglycolic acid, and derivatized celluloses such as hydroxymethylcellulose or hydroxyethylcellulose.

In some embodiments, the lipid conjugate (*e.g.*, PEG-lipid) comprises from 0.1 mol % to 2 mol % of the total lipid present in the particle. In other embodiments, the lipid conjugate (*e.g.*, PEG-lipid) comprises from 0 mol % to 20 mol % of the total lipid present in the particle.

The percentage of lipid conjugate (*e.g.*, PEG-lipid) present in the lipid particles of the invention is a target amount, and the actual amount of lipid conjugate present in the formulation may vary, for example, by $\pm$ 2 mol %. One of ordinary skill in the art will appreciate that the concentration of the lipid conjugate can be varied depending on the lipid conjugate employed and the rate at which the lipid particle is to become fusogenic.

By controlling the composition and concentration of the lipid conjugate, one can control the rate at which the lipid conjugate exchanges out of the lipid particle and, in turn, the rate at which the lipid particle becomes fusogenic. In addition, other variables including, *e.g.*, pH, temperature, or ionic strength, can be used to vary and/or control the rate at which the lipid particle becomes fusogenic. Other methods which can be used to control the rate at which the lipid particle becomes fusogenic will become apparent to those of skill in the art upon reading this disclosure. Also, by controlling the composition and concentration of the lipid conjugate, one can control the lipid particle size.

**Dilution of the Lipid Nanoparticles**

After mixing the organic lipid solution into the aqueous solution, the extent of encapsulation of dissolved drug can be enhanced if the suspension of lipid nanoparticles is further diluted prior to removal of free drug. In one embodiment the buffer can be two volumes of 15 mM Tris, 50 mM NaCl, 9% sucrose, pH 7.5 to reduce the ethanol concentration to 8.25%. In another embodiment the buffer can be three volumes of 10 mM Tris, 50 mM NaCl, 9% sucrose, pH 7.5 to reduce the ethanol concentration to 6.25%. In another embodiment the buffer can be one volume of 50mM phosphate buffer, pH 6.0 to reduce ethanol concentration to 12.5%. In another embodiment the buffer can be two volumes of 50 mM phosphate buffer, pH 6.0 to reduce ethanol concentration to 8.3%. In another embodiment the buffer can be three volumes of 50 mM phosphate buffer, pH 6.0 to reduce ethanol concentration to 6.25%. In another embodiment the buffer can be three volumes of 20 mM HEPES, 50 mM NaCl, 9% sucrose, pH 7.4 to reduce the ethanol concentration to 6.25%. In another embodiment the buffer can be 1-3 volumes of 50 mM phosphate buffer, pH 6.0, three volumes of 10 mM Tris, 50 mM NaCl, 9% sucrose, pH 7.5 to reduce the ethanol concentration to 6.25%.

The diluted lipid nanoparticles are then optionally collected in a vessel maintained at 15-20°C and allowed to incubate from a few minutes to two hours prior to a second dilution step with 10 mM Tris, 50 mM NaCl, 9% sucrose, pH 7.5 or a concentration step.

### Sample Concentration

Diluted lipid nanoparticles can be concentrated, e.g., by tangential flow filtration (TFF) using hollow fiber membranes (mPES Kros membranes, Spectrum Laboratories, Inc., Rancho Dominguez, California), optionally via a peristaltic pump or a 4-piston-diaphragm pump or a centrifugal pump (based on principle of magnetic levitation).

### Removal of Free Drug and Buffer Replacement

Concentration is followed by diafiltration, typically against 7-10 volumes of 10 mM Tris, 50 mM NaCl, 9% sucrose, pH 7.5 to remove organic solvent and unbound drug. Preferably, the diafiltration buffer is added via a heat exchanger such that product temperature is maintained at 15-20°C. The formulation is optionally further concentrated .

### Sterile Filtration

The diafiltered lipid nanoparticles are sterile filtered through a 0.2 $\mu$m sterilizing grade filter (PES) at a lipid concentration of 56-69 mg/mL.

### Sterile Fill

The filtered formulation is aseptically filled into glass vials, stoppered, capped and placed at -20 or -70 $\pm$ 5°C.

### Apparatus

The description herein provides an apparatus for carrying out the processes described above. **FIG. 2** is an example of a representative schematic of an apparatus according to one embodiment of the description herein.

The aqueous solution optionally comprising a water soluble drug is transported by an HPLC pump through tubing, e.g., comprising regions of 0.03" inner diameter (ID) PEEK tubing, 0.05" ID PEEK connector, 0.0625" silicon tubing, and 0.122" ID silicon tubing, and 0.132" ID stainless steel. The organic solution comprising lipids and optionally a hydrophobic drug is transported by an HPLC pump through tubing comprising, e.g., regions of 0.03" ID PEEK tubing, 0.02" ID PEEK connector, 0.01" ID stainless steel. The organic solution is pumped into the aqueous solution at a 90 degree angle in the mixing module. The tubing containing the rsulting mixture transports the lipid nanoparticles to a second mixing region, e.g., via a 0.25" ID polypropylene tubing which merges at a 45 degree angle with dilution buffer in the dilution area, and the diluted, optionally drug-loaded, lipid nanoparticles are collected in a stainless steel-jacketed vessel maintained at 15-20oC. The particles are further processed, e.g., by tangential flow filtration using a diaphragm or centrifugal pump.

The mixing area is in one embodiment, a mixing module in which the organic lipid solution is delivered into a stream of the aqueous solution, preferably at an angle of about 90°. A first 0.132" (3.35 mm) ID stainless steel tube transporting the aqueous solution has a hole in its wall midway between its ends. A second 0.01" ID, 0.0625" outer diameter (OD), tube is perpendicularly mounted by a filling through the hole in the wall of the first tube that allows transport of liquid from the second tube to the interior of the first tube. In preferred aspects, lipid nanoparticles of a well defined shape and reproducible size are prepared using a flow rate of the aqueous solution that is preferably three times the flow rate of the organic lipid solution. Well defined shape and reproducible size vesicles are also prepared by changing the flow rate of the fluid lines, e.g., to ensure sufficient mixing in some cases.

**FIG. 3** shows a mixing module and associated flow dynamics according to one embodiment. In comparison with prior systems, the present invention provides turbulent flow and increased shear rates. For example, the present invention advantageously provides turbulent flow ($N_{re}$ > 2000) in the mixing environment with a shear rate between about 500/s and about 3300/s at a flow rate (both flow lines) of between about 0.1 L/min and about 0.3 L/min.

The description herein also provides an apparatus having tangential flow filtration using hollow fiber membranes (e.g. mPES Kros membranes, Spectrum Laboratories, Inc., Rancho Dominguez, California) and 4-piston-diaphragm pumps or centrifugal pumps.

**Pharmaceutical Compositions**

Lipid-encapsulated drug nanoparticles described herein are useful as components in pharmaceutical compositions. These compositions will typically include a pharmaceutically acceptable carrier in addition to the liposomes. A thorough discussion of pharmaceutically acceptable carriers is available in Gennaro (2000) Remington: The Science and Practice of Pharmacy. 20th edition, ISBN: 0683306472).

Pharmaceutical compositions described herein may include the lipid nanoparticles in plain water (e.g. w.f.i.) or in a buffer, e.g., a phosphate buffer, a Tris buffer, a borate buffer, a succinate buffer, a histidine buffer, or a citrate buffer. Buffer salts will typically be included in the 5-20 mM range.

Pharmaceutical compositions as described herein may have a pH between 5.0 and 9.5 e.g. between 6.0 and 8.0.

Pharmaceutical compositions as described herein may include sodium salts (e.g. sodium chloride) to give tonicity. A concentration of $10\pm2$ mg/ml NaCl, e.g., about 9 mg/ml.

Pharmaceutical compositions as described herein may include metal ion chelators. These can prolong drug stability by removing ions which can accelerate phosphodiester hydrolysis, e.g., one or more of EDTA, EGTA, BAPTA, or pentetic acid. Such chelators are preferably at between 10-500 $\mu$M, e.g., 0.1 mM. A citrate salt, such as sodium citrate, can also act as a chelator, while advantageously also providing buffering activity.

Pharmaceutical compositions as described herein preferably have an osmolality of between 200 mOsm/kg and 400 mOsm/kg, e.g., between 240-360 mOsm/kg, or between 290-310 mOsm/kg.

Pharmaceutical compositions as described herein preferably include one or more preservatives, such as thiomersal or 2-phenoxyethanol. Mercury-free compositions are most preferred.

Pharmaceutical compositions as described herein are preferably sterile. Pharmaceutical compositions as described herein are preferably non-pyrogenic e.g. containing < 1 EU (endotoxin unit, a standard measure) per dose, and preferably <0.1 EU per dose.

Pharmaceutical compositions as described herein are preferably gluten free. Pharmaceutical compositions as described herein may be prepared in unit dose form. In some embodiments a unit dose may have a volume of between 0.1-1.0 ml e.g. about 0.5 ml.

The compositions may be prepared as injectables, either as solutions or suspensions. The composition may be prepared for pulmonary administration e.g. by an inhaler, using a fine spray. The composition may be prepared for nasal, aural or ocular administration e.g. as spray or drops.

Pharmaceutical compositions comprise an immunologically effective amount of lipid nanoparticles, as well as any other components, as needed.

Pharmaceutical compositions as described herein are also suitable for a delivery device, e.g., syringe, nebulizer, sprayer, inhaler, or dermal patch, which can be used to administer the composition to a vertebrate subject.

**EXAMPLES**

**Example 1: Effect of flow rate on particle size**

A reservoir containing an aqueous buffer and another reservoir with an ethanolic lipid solution comprising total lipid at a concentration of 49.4 mg/mL were prepared, as shown in FIG. 1.

The lipid solution was pumped from the reservoir through a mixing module by an HPLC pump through tubing having a 0.01" lipid inlet at flow rates from 25 to 87.5 ml/min for lipid. The aqueous buffer solution was pumped from the reservoir through a module by an HPLC pump through tubing having 0.132" inlet. The flow rate of the aqueous buffer was three-times the flow rate of the lipid solution, and the output of the mixing was a 25% EtOH solution. The output solution was further diluted in buffer, and and ethanol was removed by Tangential Flow Filtration (TFF). Figs. 1, 2, and 3 schematically shows the overall process.

Various properties of the lipid nanoparticles produced were measured, including average particle size, (nm), polydispersity index (PDI). Formulation yields were > 80% for all conditions tested. The results are shown in Table 5.

**Table 5: Varying flow rates during mixing**

| Flow Rate, ml/min | | | Average Particle Size (nm) | PDI | | Yield (%) | |
|---|---|---|---|---|---|---|---|
| Lipid | Buffer | Total | | | | | |
| 25 | 75 | 100 | 106.9 | 0.09 | | 76 | |
| 50 | 150 | 200 | 75.7 | 0.08 | | 82 | |
| 75 | 225 | 300 | 71.2 | 0.07 | | 85 | |
| 87.5 | 262.5 | 350 | 66.4 | 0.08 | | 80 | |

The results showed that increasing the flow rate of the lipid and buffer solutions decreased the average particle size while maintaining the PDI at < 0.1

**Example 2: Process Scalability and Reproducibility**

The conditions of Example 1 were followed using a combined flow rate of 300 ml/min for a batch volume from 1 liter to 220 liters. The results are shown in Table 6.

**Table 6: Scalability and reproducibility of physicochemical properties**

| Batch Volume (L) | Average Particle Size (nm) | PDI | | |
|---|---|---|---|---|
| 1.8 | 75.4 | 0.07 | | |
| 3.5 | 73.6 | 0.04 | | |
| 7.3 | 73.7 | 0.04 | | |
| 22 | 76.1 | 0.04 | | |
| 110 | 76.0 | 0.06 | | |
| 220 | 76.0 | 0.06 | | |
| 220 | 75.3 | 0.05 | | |
| 220 | 75.0 | 0.04 | | |

The results demonstrate that the process disclosed herein using turbulence can be scaled from 1.8 liters to 220 liters. The key physicochemical and biological properties are unchanged through the scaling. FIG. 5 shows that particles produced are uniform in size at 220 L batch.

The scalability of the process disclosed herein was measured using the conditions disclosed in Example 1 by measuring particle size and PDI. The results are shown in FIG. 4A. The results demonstrate that the size of the particles remains between 70 and 80 nm during the scale up.

The reproducibility of the process was measured by preparing several batches of lipid nanoparticles independently. The results are shown in FIG. 4B. The results demonstrate inter-batch reproducibility, in which particle size of 70-80 nm was obtained. Final formulation yields were > 80% across all scales from 1.8-220 L.

**Example 3: Effect of module size**

The conditions of Example 1 were followed. The effect of varying the module inlet diameters were measured varying the flow rate from 40 to 600 ml/min, total, as shown with results in Table 9. Inlet pressure was monitored, and the size PDI of the resulting lipid nanoparticles were measured.

**Table 9 Varying module inlet diameters**

| Batch ID | inlet ID (inch) | | inlet pressure (psi) | | Flow Rate (ml/min) | | | Average Particle Size (nm) | PDI |
|---|---|---|---|---|---|---|---|---|---|
| | Lipid | Buffer | Lipid | Buffer | Lipid | Buffer | Total | | |
| 10 | 0.005 | | 209 | 0 | 10 | 30 | 40 | 131 | 0.09 |
| 11 | | | 386 | 0 | 15 | 45 | 60 | 94.07 | 0.14 |
| 12 | | | 622 | 0 | 20 | 60 | 80 | 70.83 | 0.10 |
| 13 | 0.007 | 0.132 | 172 | 0 | 25 | 75 | 100 | 97.74 | 0.12 |
| 14 | | | 244 | 3 | 30 | 90 | 120 | 84.88 | 0.14 |
| 15 | | | 439 | 8 | 35 | 105 | 140 | 72.62 | 0.12 |
| 16 | | | 617 | 14 | 40 | 120 | 160 | 64.28 | 0.09 |
| 17 | 0.01 | | 5 | 0 | 25 | 75 | 100 | 106.9 | 0.09 |
| 18 | | | 96 | 27 | 50 | 150 | 200 | 75.7 | 0.08 |
| 19 | | | 343 | 75 | 75 | 225 | 300 | 69.06 | 0.11 |
| 20 | | | 477 | 101 | 87.5 | 262.5 | 350 | 66.38 | 0.08 |
| 21 | 0.02 | | 0 | 100 | 75 | 225 | 300 | 135.6 | 0.08 |
| 22 | | | 2 | 170 | 100 | 300 | 400 | 119.6 | 0.12 |
| 23 | | | 56 | 345 | 150 | 450 | 600 | 100.4 | 0.16 |
| 24 | 0.005 | 0.1 | 186 | 0 | 10 | 30 | 40 | 136.8 | 0.11 |
| 25 | | | 350 | 0 | 15 | 45 | 60 | 104.6 | 0.16 |
| 26 | | | 566 | 0 | 20 | 60 | 80 | 81.24 | 0.13 |

The results show that without changing the composition, only by changing the lipid and/or aqueous buffer inlets' IDs of the mixing module can generate different sizes of nanoparticles with > = 80% yields.

**Claims**

1. A method of producing a lipid nanoparticle optionally comprising a desired drug, comprising the steps
pumping an aqueous solution optionally comprising a water-soluble drug through a 1st tube having an inner diameter (ID) of at least 0.1 ";
pumping an ethanol solution comprising lipids and optionally a hydrophobic drug through a 2nd tube having an ID of at least 0.005" at one third the flow rate of the 1st solution through the 1st tube; and
mixing the ethanol solution with the aqueous solution by introducing the ethanol solution into the aqueous solution in the 1st tube flowing past the 2nd tube within a mixing module consisting of the 2nd tube perpendicularly joined to the 1st tube,
wherein the mixing produces an output comprising a turbulent flow of the resulting mixed solution comprising spontaneously formed lipid nanoparticles in 25% ethanol, and wherein the lipid nanoparticles have a bilayer structure.

2. The method of claim 1, wherein the output has a flow rate of at least 200 ml/min.

3. The method according to claim 1 or 2, wherein the flow rate of the output has a Reynolds number of at least 2000.

4. The method according to any of claims 1 to 3, wherein the aqueous solution is pumped with a back pressure of at least 10 psi, 25 psi, 50 psi, 75 psi, or 100 psi, at a flow rate of at least 30 ml/min, 45 ml/min, 60 ml/min, 75 ml/min, 90 ml/min, 105 ml/min, 120 ml/min, 150 ml/min, 225 ml/min, 262.5 ml/min, 300 ml/min, or 450 ml/min.

5. The method according to any of claims 1 to 4, wherein the ethanol solution is pumped with a back pressure of at least 40 psi, 80 psi, 150 psi, 300 psi, or 400 psi, at a flow rate of at least 10 ml/min, 15 ml/min, 20 ml/min, 25 ml/min, 30 ml/min, 35 ml/min, 40 ml/min, 50 ml/min, 60 ml/min, or 75 ml/min, 87.5 ml/min, 100 ml/min, or 150 ml/min.

6. The method according to any of claims 1 to 5, wherein the aqueous solution is pumped through the 1st tube by a 1st HPLC pump and the ethanol solution is pumped through the 2nd tube by a 2nd HPLC pump, wherein the 1st tube has an ID of 0.132" and the 2nd tube has an ID of 0.007", 0.01 ", or 0.02".

7. The method according to any of claims 1 to 6, wherein the aqueous and ethanol solutions are maintained at 15-20°C.

8. The method according to any of claims 1 to 7, further comprising pumping a dilution buffer through a 3rd tube, and mixing the dilution buffer with the output solution by introducing the dilution buffer to the output solution via a Y connector to produce a diluted output solution.

9. The method of claim 8, wherein the dilution buffer comprises
15 mM Tris, 50 mM NaCl, 9% sucrose, pH 7.5; or
10mM Tris, 50mM NaCl, 9% sucrose, pH 7.5; or
50mM phosphate, pH 6.0; or
20mM HEPES, 50mM NaCl, 9% sucrose, pH 7.4; or
50mM HEPES, 50mM NaCl, 9% sucrose, pH 7.5.

10. The method of claim 8 or 9, wherein the diluted output solution comprises 6.25% ethanol; 8.25% ethanol; 8.3% ethanol; or 12.5% ethanol.

11. The method according to any of claims 8 to 10, wherein the 3rd tube has an ID of 0.25" and the dilution buffer is pumped through the 3rd tube at a flow rate of 400-900 mL/min.

12. The method according to any of claims 1 to 11, wherein the lipid nanoparticles have a bilayer structure and an average particle size of less than 70 nm, 80 nm, 90 nm, or 100 nm.

13. The method according to any of claims 1 to 12, wherein the lipid nanoparticles have a polydispersity index (PDI) of less than 0.05, 0.07, or 0.09, and/or wherein the variability of average particle size between batches is less than 10%.

14. An apparatus for producing lipid-encapsulated RNA nanoparticles, comprising
a 1st tube having an inner diameter (ID) of at least 0.1" connected at one end to a 1st HPLC pump and at the other end to a mixing module, wherein the 1st HPLC pump is configured to pump an aqueous solution optionally comprising a water-soluble drug through the 1st tube at a flow rate of at least 150 ml/min;
a 1st reservoir connected to the 1st HPLC pump, wherein the 1st reservoir contains the aqueous solution optionally comprising a water-soluble drug;
a 2nd tube having an ID of at least 0.005" connected at one end to a 2nd HPLC pump and at the other end to the mixing module, wherein the 2nd HPLC pump is configured to pump an ethanol solution comprising lipid and optionally a hydrophobic drug through the 2nd tube at a flow rate greater than 50 ml/min, and wherein the 2nd tube is perpendicularly joined to the 1st tube at the mixing module;
a 2nd reservoir connected to the 2nd HPLC pump, wherein the 2nd reservoir contains the ethanol solution optionally comprising a hydrophobic drug;
wherein the apparatus is configured to mix the ethanol solution with the aqueous solution by introducing the ethanol solution into the aqueous solution in a region within the mixing module wherein the mixing produces an output comprising a turbulent flow of the mixed solution comprising spontaneously formed lipid nanoparticles in 25% ethanol.

15. The apparatus of claim 14, wherein at the mixing module the 2nd tube extends through a wall of the 1st tube and partly into the interior of the 1st tube, or wherein at the mixing module the 2nd tube extends up to a wall of the 1st tube and joins the 1st tube.

16. The apparatus of any of claim 14 or 15, further comprising a Y connector for introducing a dilution buffer through a 3rd tube to the output solution to produce a diluted output solution, wherein the Y-connector is preferably at an angle of 40, 45, or 50 degrees.

**Fig. 1**

```
┌──────────────────┐        ┌──────────────────┐
│   Weigh Lipids   │        │    Weigh Drug    │
└────────┬─────────┘        └────────┬─────────┘
         │                           │
┌────────┴─────────┐        ┌────────┴─────────┐
│  Dissolution in  │        │ Dilution in Buffer│
│     Ethanol      │        │                  │
└────────┬─────────┘        └────────┬─────────┘
         │                           │
┌────────┴─────────┐        ┌────────┴─────────┐
│ Filtration 0.2μm │        │ Filtration 0.2μm │
└────────┬─────────┘        └────────┬─────────┘
         │                           │
┌──────────────────┐        ┌──────────────────┐
│ In-Process Control│       │ In-Process Control│
│ Lipid Concentration│      │ Drug Concentration│
└──────────────────┘        └──────────────────┘
         └─────────────┬─────────────┘
              ┌────────┴─────────┐
              │ Mixing and Dilution│
              │  to Form Particles │
              └────────┬─────────┘
┌──────────────────┐   │
│ In-Process Control│───┤
│     (PS, PDI)    │   │
└──────────────────┘   │
              ┌────────┴─────────┐
              │ Purification by TFF│
              └────────┬─────────┘
┌──────────────────┐   │
│ In-Process Control│───┤
│     (PS, PDI)    │   │
└──────────────────┘   │
              ┌────────┴─────────┐
              │ Filtration 0.2μm │
              └────────┬─────────┘
┌──────────────────┐   │
│ In-Process Control│───┤
│  (PS, PDI, Drug) │   │
└──────────────────┘   │
              ┌────────┴─────────┐
              │ Drug Concentration│
              │    Adjustment    │
              └────────┬─────────┘
              ┌────────┴─────────┐
              │ Filtration 0.2μm │
              └────────┬─────────┘
              ┌────────┴─────────┐
              │Fill, Finish and Freeze│
              └──────────────────┘
```

**Fig. 2**

**Fig. 3**

Europäisches
Patentamt

European
Patent Office

Office européen
des brevets

**EUROPEAN SEARCH REPORT**

Application Number

EP 19 16 3780

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2010/078045 A2 (ONCOTHYREON INC [US]; DUPUIT ROBERT A [CA]; REILLEY WILLIAM J [US]) 8 July 2010 (2010-07-08)<br>* page 9, line 13 - line 27 *<br>* page 10, line 1 - line 2 *<br>* claims 1-5 *<br>* figures 1-3 * | 1-16 | INV.<br>A61K9/127<br>A61K38/00<br>A61K9/51 |
| Y | US 2019/029959 A1 (COSTA ANTONIO [US] ET AL) 31 January 2019 (2019-01-31)<br>* figures 6,7 *<br>* paragraph [0091] *<br>* examples 1-3 * | 1-16 | |
| Y | US 2018/263918 A1 (DEROSA FRANK [US] ET AL) 20 September 2018 (2018-09-20)<br>* example 2 *<br>* claims 48-50 * | 1-16 | |
| Y | US 4 895 452 A (YIOURNAS COSTAS [US] ET AL) 23 January 1990 (1990-01-23)<br>* figure 1 *<br>* column 3, line 39 - line 45 *<br>* column 5, line 42 - line 47 *<br>* claims 1-4 *<br>* column 7, line 30 - line 33 * | 1-16 | TECHNICAL FIELDS SEARCHED (IPC)<br><br>A61K |
| Y | US 2012/021042 A1 (PANZNER STEFFEN [DE] ET AL) 26 January 2012 (2012-01-26)<br>* figures 1-3 *<br>* example 4 * | 1-16 | |

-/--

The present search report has been drawn up for all claims

| Place of search | Date of completion of the search | Examiner |
|---|---|---|
| Munich | 28 August 2019 | Sindel, Ulrike |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EUROPEAN SEARCH REPORT**

Europäisches
Patentamt
European
Patent Office
Office européen
des brevets

Application Number

EP 19 16 3780

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | IGOR V. ZHIGALTSEV ET AL: "Bottom-Up Design and Synthesis of Limit Size Lipid Nanoparticle Systems with Aqueous and Triglyceride Cores Using Millisecond Microfluidic Mixing", LANGMUIR, vol. 28, no. 7, 21 February 2012 (2012-02-21), pages 3633-3640, XP055150435, ISSN: 0743-7463, DOI: 10.1021/la204833h * figure 1 * * page 3634, column 2, paragraph 2 * * page 3635, column 1, paragraph 4 * | 1-16 | |
| Y | US 2016/243255 A1 (NECHEV LUBOMIR [US] ET AL) 25 August 2016 (2016-08-25) * paragraph [0025] - paragraph [0030] * * example 7 * | 1-16 | |
| Y | US 2017/196809 A1 (BOWMAN KEITH A [US] ET AL) 13 July 2017 (2017-07-13) * figures 2, 3 * * paragraph [0269] - paragraph [0273] * * paragraph [0302] - paragraph [0345] * | 1-16 | TECHNICAL FIELDS SEARCHED (IPC) |

The present search report has been drawn up for all claims

| Place of search | Date of completion of the search | Examiner |
|---|---|---|
| Munich | 28 August 2019 | Sindel, Ulrike |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 3 711 749 A1**

**ANNEX TO THE EUROPEAN SEARCH REPORT
ON EUROPEAN PATENT APPLICATION NO.**

EP 19 16 3780

28-08-2019

| Patent document cited in search report | | | Publication date | Patent family member(s) | | | Publication date |
|---|---|---|---|---|---|---|---|
| WO 2010078045 | A2 | | 08-07-2010 | AU | 2009333177 | A1 | 07-07-2011 |
| | | | | BR | PI0923001 | A2 | 18-09-2018 |
| | | | | CA | 2747182 | A1 | 08-07-2010 |
| | | | | CN | 102256595 | A | 23-11-2011 |
| | | | | CN | 105935352 | A | 14-09-2016 |
| | | | | EA | 201100829 | A1 | 28-02-2012 |
| | | | | EP | 2367532 | A2 | 28-09-2011 |
| | | | | JP | 5895030 | B2 | 30-03-2016 |
| | | | | JP | 2012512260 | A | 31-05-2012 |
| | | | | JP | 2014224127 | A | 04-12-2014 |
| | | | | KR | 20110094114 | A | 19-08-2011 |
| | | | | SG | 172257 | A1 | 28-07-2011 |
| | | | | US | 2012034294 | A1 | 09-02-2012 |
| | | | | US | 2013330398 | A1 | 12-12-2013 |
| | | | | US | 2015315217 | A1 | 05-11-2015 |
| | | | | WO | 2010078045 | A2 | 08-07-2010 |
| US 2019029959 | A1 | | 31-01-2019 | CA | 2977827 | A1 | 22-09-2016 |
| | | | | CN | 107427791 | A | 01-12-2017 |
| | | | | EP | 3271057 | A1 | 24-01-2018 |
| | | | | US | 2019029959 | A1 | 31-01-2019 |
| | | | | WO | 2016149625 | A1 | 22-09-2016 |
| US 2018263918 | A1 | | 20-09-2018 | AU | 2015283954 | A1 | 22-12-2016 |
| | | | | BR | 112016030852 | A2 | 16-01-2018 |
| | | | | CA | 2953265 | A1 | 07-01-2016 |
| | | | | CN | 106456547 | A | 22-02-2017 |
| | | | | EP | 3164112 | A1 | 10-05-2017 |
| | | | | JP | 2017520551 | A | 27-07-2017 |
| | | | | US | 2016038432 | A1 | 11-02-2016 |
| | | | | US | 2017348244 | A1 | 07-12-2017 |
| | | | | US | 2018263918 | A1 | 20-09-2018 |
| | | | | WO | 2016004318 | A1 | 07-01-2016 |
| US 4895452 | A | | 23-01-1990 | AU | 614598 | B2 | 05-09-1991 |
| | | | | BR | 8907293 | A | 12-03-1991 |
| | | | | CA | 1320400 | C | 20-07-1993 |
| | | | | DE | 68910969 | D1 | 05-01-1994 |
| | | | | DE | 68910969 | T2 | 23-06-1994 |
| | | | | EP | 0406273 | A1 | 09-01-1991 |
| | | | | JP | 2542271 | B2 | 09-10-1996 |
| | | | | JP | H03504101 | A | 12-09-1991 |
| | | | | US | 4895452 | A | 23-01-1990 |
| | | | | WO | 8907929 | A1 | 08-09-1989 |
| US 2012021042 | A1 | | 26-01-2012 | US | 2012021042 | A1 | 26-01-2012 |

EPO FORM P0459

For more details about this annex : see Official Journal of the European Patent Office, No. 12/82

page 1 of 2

**ANNEX TO THE EUROPEAN SEARCH REPORT
ON EUROPEAN PATENT APPLICATION NO.**                    EP 19 16 3780

This annex lists the patent family members relating to the patent documents cited in the above-mentioned European search report.
The members are as contained in the European Patent Office EDP file on
The European Patent Office is in no way liable for these particulars which are merely given for the purpose of information.

28-08-2019

| Patent document cited in search report | Publication date | Patent family member(s) | Publication date |
|---|---|---|---|
| | | US 2014056970 A1 | 27-02-2014 |
| US 2016243255 A1 | 25-08-2016 | CA 2921161 A1 | 02-04-2015 |
| | | EP 3049066 A2 | 03-08-2016 |
| | | US 2016243255 A1 | 25-08-2016 |
| | | WO 2015048020 A2 | 02-04-2015 |
| US 2017196809 A1 | 13-07-2017 | CN 106794141 A | 31-05-2017 |
| | | EP 3169309 A1 | 24-05-2017 |
| | | JP 2017523965 A | 24-08-2017 |
| | | US 2017196809 A1 | 13-07-2017 |
| | | WO 2016010840 A1 | 21-01-2016 |

EPO FORM P0459

For more details about this annex : see Official Journal of the European Patent Office, No. 12/82

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0105373 A
- US 20040142025 A
- US 6843942 B
- US 9005654 B
- US 9404127 B
- US 9593077 B
- US 9365610 B
- US 9567296 B
- US 9580711 B
- US 9670152 B
- US 20170190661 A
- US 20170114010 A

**Non-patent literature cited in the description**

- Fluid and Particle Dynamics. **TILTON.** PERRY'S CHEMICAL ENGINEERS' HANDBOOK. 2008, 6-51
- **POLING.** Physical and Chemical Data. PERRY'S, 2-117
- **GENNARO.** Remington: The Science and Practice of Pharmacy. 2000